# EUROPEAN PATENT APPLICATION

(11) **EP 3 851 512 A1**
(43) Date of publication of application: **21.07.2021**
(21) Application number: 19877491.1
(22) Date of filing: 27.09.2019
(51) Int. Cl.: C12M 1/00

(54) **CULTURING APPARATUS**

(30) Priority: 26.10.2018 JP 2018201702
(71) Applicant: PHC Holdings Corporation, Tokyo 105-8433 (JP)
(72) Inventor: ONDA, Megumi, Toon-shi, Ehime (JP); SAWAI, Akito, Toon-shi, Ehime (JP)
(74) Representative: Grünecker Patent- und Rechtsanwälte PartG mbB
(86) International application number: PCT/JP2019/038346
(87) International publication number: WO 2020/085006

(57) **Abstract**

This culturing apparatus is provided with a humidifying pan which is disposed in a culturing space and in which a liquid for humidification is retained, a first heater for heating the humidifying pan; and a second heater for controlling the temperature of the culturing space, the first heater and the second heater being separately provided.

## Description

### Technical Field

The present invention relates to a culture apparatus.

### Background Art

In a culture apparatus (incubator) for incubating a culture such as a cell or a microorganism, it is necessary to control the humidity in a culture space at a humidity suitable for incubating the culture (see, for example, Patent Literature (hereinafter, referred to as "PTL") 1)

Humidification of the culture space is performed using a humidification tray placed on the bottom wall of the culture space. Specifically, the culture space is humidified by heating the humidification tray with a heater wire disposed on the bottom wall to evaporate water in the humidification tray.

The humidification tray is heated by a portion of the heater wire used to adjust the temperature in the culture space, specifically, by the portion located at the periphery of the humidification tray. That is, a single heater wire is used for both temperature adjustment and humidity adjustment.

### Citation List

### Patent Literature

PTL 1
Japanese Patent Application Laid-Open No. 2017-201886

### Summary of Invention

### Technical Problem

As described above, since the single heater wire is used for both temperature adjustment and humidity adjustment in the conventional culture apparatus, the capacity of the heater wire is increased, and the following problems have occurred.
(1) Fine control on the output of the heater wire is difficult, and it is thus difficult to finely control the humidity.
(2) The responsiveness of the heater wire is low, and is it thus impossible to quickly increase the humidity in the culture space to a range suitable for incubation of the culture.

The present invention has been devised to solve such problems, and aims to provide a culture apparatus capable of finely and quickly adjusting the humidity in a culture space.

### Solution to Problem

In order to solve the above-mentioned conventional problems, a culture apparatus of the present invention includes: a humidification tray that is disposed in a culture space and stores a humidification liquid; a first heater that heats the humidification tray; and a second heater for controlling a temperature in the culture space, in which the first heater and the second heater are separately provided.

### Advantageous Effects of Invention

According to the present invention, it is possible to finely and quickly adjust the humidity in the culture space.

### Brief Description of Drawings

FIG. 1 illustrates a schematic longitudinal section of a culture apparatus of an embodiment of the present invention as seen from the right side;
FIG. 2 is a schematic plan view illustrating a pattern of heater wires; and
FIG. 3 is a schematic functional block diagram illustrating a principal part of a control configuration of the culture apparatus of an embodiment of the present invention.

### Description of Embodiments

Hereinafter, a culture apparatus according to an embodiment of the present invention will be described with reference to the accompanying drawings. The following embodiments are merely illustrative, and various modifications and/or applications of techniques which are not specified in the following embodiments are not excluded. In addition, the configurations of the embodiments can be variously modified and implemented without departing from the spirit thereof. Further, the configurations of the embodiments can be selected as necessary, or can be appropriately combined.

In the following description, the side of the culture apparatus which the user faces during usage of the culture apparatus (the side with below-described outer door 3a and inner door 3b) is referred to as "front" and the side opposite to the front is referred to as "rear." In addition, the left and right are defined with reference to the case of viewing from the front to the rear.

Note that, in all the figures for explaining the embodiments, the same elements are denoted by the same reference numerals in principle, and the description thereof may be omitted.

### [1. Configuration]

### [1-1. Overall Configuration]

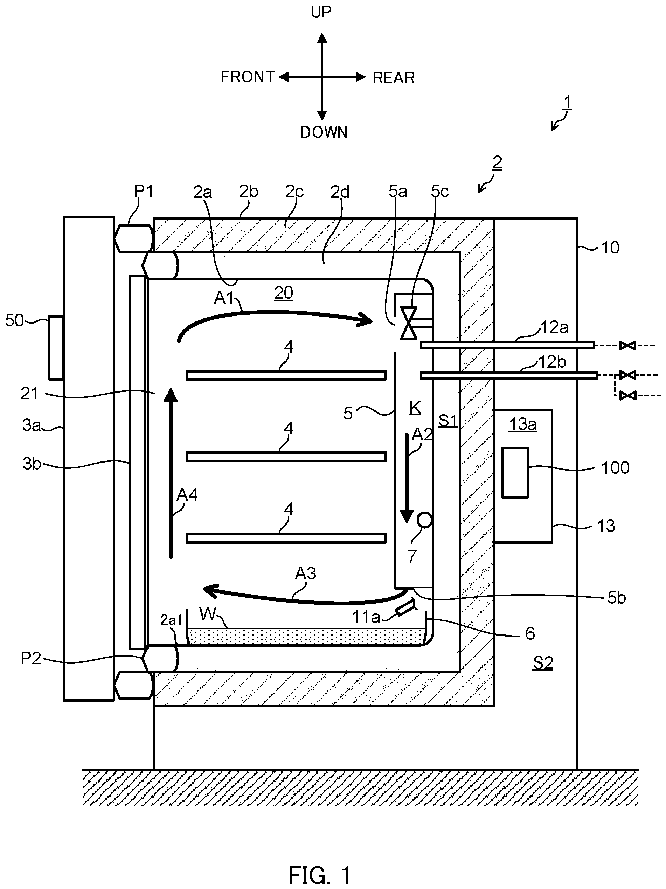

A description will be given of an entire culture apparatus of an embodiment of the present invention with reference to FIG. 1. FIG. 1 illustrates a schematic longitudinal section of the culture apparatus of an embodiment of the present invention as seen from the right side.

Culture apparatus 1 illustrated in FIG. 1 is an apparatus for incubating a culture such as a cell or a microorganism. Culture apparatus 1 is configured to include substantially box-shaped heat insulation box 2 having culture space 20 formed inside and opening 21 formed in the front surface, outer door 3a and inner door 3b for opening and closing opening 21. Culture space 20 is vertically compartmentalized by a plurality of (here, three) shelves 4. Packing P1 is disposed on the outer edge of outer door 3a.

The temperature, humidity, O₂ (oxygen) concentration, and CO₂ (carbon dioxide) concentration are controlled to be maintained within respective suitable ranges such that an environment of the culture space becomes a suitable environment for incubating the culture.

Heat insulation box 2 includes substantially box-shaped inner box 2a having culture space 20 formed inside, and substantially box-shaped outer box 2b that covers the outside of inner box 2a.

Outer box 2b is provided, on its inner surface side, with heat insulation material 2c. Space S1 is formed between the inner surface of heat insulation material 2c of outer box 2b and the outer surface of inner box 2a in such a manner as to cover the upper, lower, left, right, and rear sides of inner box 2a. This space S1 is filled with air; the air layer (so-called air jacket) 2d is formed in space S1. Space S1 has an opening in the front, and this opening is sealed with packing P2.

In culture space 20, vertically extending duct 5 is disposed on the rear surface of inner box 2a. Gas passage K is formed inside duct 5. Circulation blower 5c is disposed in gas passage K. By operating circulation blower 5c, air in culture space 20 is sucked through suction port 5a formed in an upper portion of duct 5, and this air is blown out to culture space 20 through blow-out port 5b formed in a lower portion of duct 5. Thus, forced circulation of the air as indicated by arrows A1, A2, A3, and A4 takes place.

In addition, ultraviolet lamp 7 and gas supply pipes 12a and 12b for supplying, to culture space 20, adjustment gases (O₂ gas, N₂ gas, and CO₂ gas) for adjusting O₂ gas concentration and CO₂ gas concentration in culture space 20 are installed within duct 5. Ultraviolet lamp 7 sterilizes water W in humidification tray 6 described later.

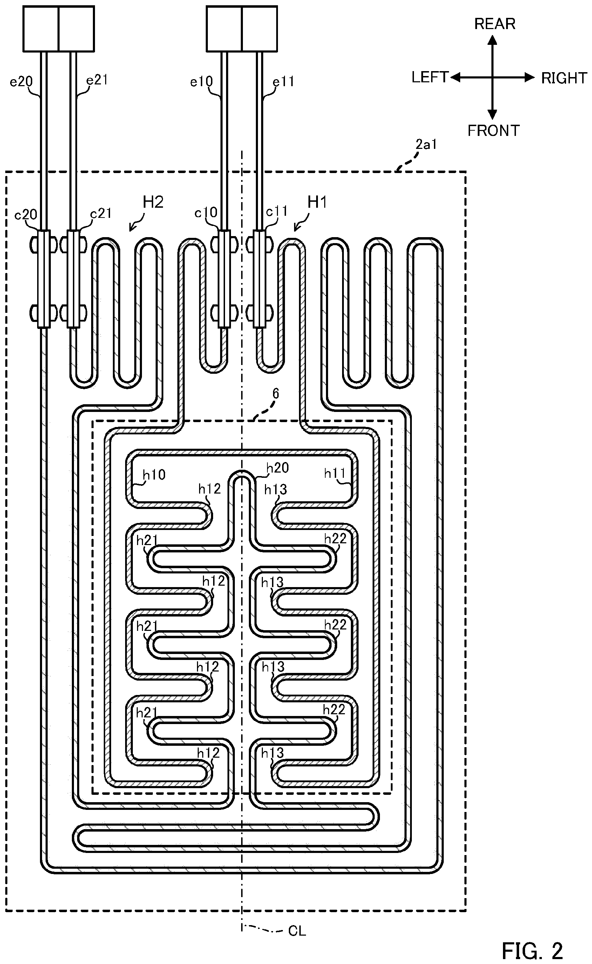

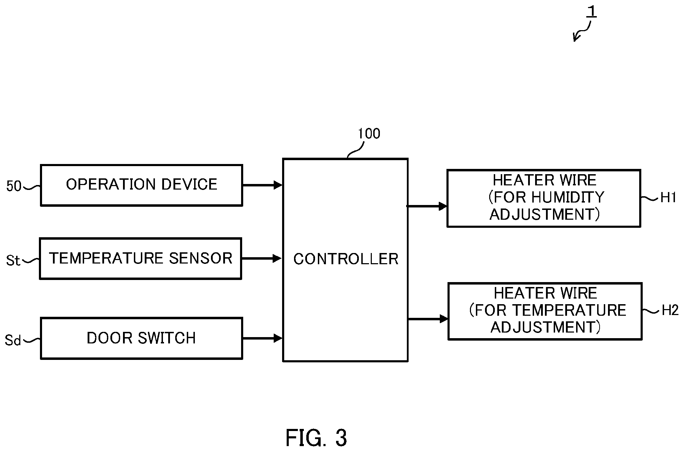

In addition, humidification tray 6 for storing water W for humidification (hereinafter referred to as "humidification water W") is installed between the lower portion of duct 5 and bottom wall 2a1 of inner box 2a. Humidification tray 6 is heated by a linear heater (hereinafter referred to as "heater wire") H1 (see FIGS. 2 and 3) disposed on the lower surface of bottom wall 2a1 of inner box 2a. Heated by heater wire HI, humidification water W is evaporated to humidify culture space 20. That is, heater wire H1 is a humidity adjustment heater.

In addition, heater wires H2 (see FIGS. 2 and 3) for temperature adjustment, that is, for controlling the temperature in culture space 20, are installed respectively on the rear surfaces (surfaces on the side of outer box 2b) of the right side wall, the left side wall, the rear wall, the top wall, and the bottom wall of inner box 2a. Note that heater wires H2 are being energized and generating heat, in principle, during the operation of culture apparatus 1.

These heater wire H1 and heater wires H2 are provided separately, and the energization rate of the heater wires are individually controlled by controller 100 to control the outputs (heating forces) individually.

In addition, culture apparatus 1 receives instructions to start and stop culture apparatus 1 and/or inputs of various setting values of culture space 20 from operation device 50 disposed on outer door 3a. The various setting values of culture space 20 are a set temperature, set humidity, set concentration of O₂ gas, set concentration of CO₂ gas, and the like. Controller 100 controls the temperature, humidity, O₂ concentration, CO₂ concentration, and the like in culture space 20 such that the temperature, humidity, O₂ concentration, CO₂ concentration, and the like achieve the above-mentioned set values.

The rear and bottom surfaces of outer box 2b of heat insulation box 2 are covered with cover 10. The space between the rear surface of outer box 2b and cover 10 forms mechanical room S2 for disposing various equipment therein. Electrical box 13 is disposed in mechanical room S2. Controller 100 and other electrical components (not illustrated) are housed in inside 13a of electrical box 13.

Further, the tip of dew condensation member 11a is inserted into culture space 20. Dew condensation member 11a is cooled by a Peltier element (not illustrated). Accordingly, condensation water is generated on the surface of dew condensation member 11a in culture space 20. Generation of the condensation water makes it possible to reduce the humidity in culture space 20 to control the humidity within an appropriate range. Note that, the condensation water generated on the surface of dew condensation member 11a drips from the tip of dew condensation member 11a into humidification tray 6.

### [1-2. Heater Pattern]

A description will be given of a pattern of heater wires H1 and H2 (heater pattern) with reference to FIG. 2. FIG. 2 schematically illustrates the pattern of heater wires H1 and H2 as seen from above. In FIG. 2, for convenience, bottom wall 2a1 of inner box 2a and humidification tray 6 are indicated by broken lines.

As illustrated in FIG. 2, heater wire H1 is connected to power cables e10 and e11 via crimp terminals c10 and c11. Likewise, heater wire H2 is connected to power cables e20 and e21 via crimp terminals c20 and c21.

Humidity adjustment heater wire H1 repeatedly meanders in the front-rear direction (second direction) or the left-right direction (first direction) while forming U-shapes, and is disposed mostly below humidification tray 6. Temperature adjustment heater wire H2 repeatedly meanders similarly in the front-rear direction or the left-right direction while forming U-shapes, and is disposed substantially over entire bottom wall 2a1.

Focusing on the lower side of humidification tray 6, a portion of temperature adjustment heater wire H2 disposed below humidification tray 6 includes, in the middle in the left-right direction, U-turn portion h20 (first portion) extending in the front-rear direction. A plurality of U-turn portions h21 (second portions) extending in the left direction and a plurality of U-turn portions h22 (second portions) extending in the right direction are connected to U-turn portion h20. U-turn portions h21 and h22 have the same arrangements in the front-rear direction. U-turn portions h21 and h22 form pairs and a plurality of pairs of U-turn portions h21 and h22 are disposed to be spaced from one another in the front-rear direction.

Likewise, focusing on the lower side of humidification tray 6, a large portion of humidity adjustment heater wire H1 disposed below humidification tray 6 includes U-turn portions h10 and h11 (third portions) extending in the front-rear direction respectively at the left and right side edges of humidification tray 6. A plurality of U-turn portions h12 and h13 (fourth portions) extending to the vicinity of center line CL of humidification tray 6 in the left-right direction are connected to U-turn portions h10 and h11, respectively. Specifically, a plurality of U-turn portions h12 extending in the right direction are connected to left U-turn portion h10, and a plurality of U-turn portions h13 extending in the left direction are connected to right U-turn portion h11. U-turn portions h12 and h13 have the same arrangements in the front-rear direction. U-turn portions h12 are disposed between and ahead of or behind U-turn portions h21 of temperature adjustment heater wire H2, and U-turn portions h13 are disposed between and ahead of or behind U-turn portions h22 of temperature adjustment heater wire H2. In other words, U-turn portions h12 and h21 are alternately arranged in the front-rear direction, and likewise, U-turn portions h13 and h22 are alternately arranged in the front-rear direction.

### [2. Control Configuration]

Hereinafter, a principal part of a control configuration of culture apparatus 1 of an embodiment of the present invention will be described with reference to FIG. 3.

FIG. 3 is a schematic functional block diagram illustrating the principal part of the control configuration of culture apparatus 1 of an embodiment of the present invention.

As illustrated in FIG. 3, operation device 50, temperature sensor St, and door switch Sd input control signals to controller 100. Further, controller 100 outputs a control command to heater wires H1 and H2.

Operation device 50 receives various inputs. Specifically, operation device 50 receives an operation of starting (turning on the power of) culture apparatus 1, an operation of stopping (turning off the power of) culture apparatus 1, and an operation of inputting various settings such as set temperature T, set humidity, set CO₂ concentration x [%], and set O₂ concentration y [%] in culture space 20. Operation device 50 inputs these as control signals to controller 100. Note that, the function of setting the humidity may be omitted.

Temperature sensor St detects the temperature in culture space 20, and outputs the detected temperature to controller 100 as a control signal.

Door switch Sd detects the opening and closing of outer door 3a. Door switch Sd may output a closing signal in the closed state of outer door 3a, and output a closing signal in the open state of outer door 3a, or may output a signal only in either of the closed state and the open state of outer door 3a.

Further, controller 100 controls the output of temperature adjustment heater wire H2 based on the temperature in culture space 20 detected by temperature sensor St (hereinafter referred to as "temperature detection value"). Specifically, the energization rate of heater wire H2 is controlled such that the heating force of heater wire H2 increases when the temperature detection value is lower than set temperature T by a predetermined value or by a value greater than the predetermined value, and such that the heating force of heater wire H2 decreases when the temperature detection value is higher than set temperature T by a predetermined value or by a value greater than the predetermined value.

In addition, when it is necessary to increase the humidity in culture space 20 quickly, controller 100 increases the heating power (output) of heater wire H1 by increasing the energization rate of humidity adjustment heater wire H1.

Further, controller 100 determines that the humidity in the culture space needs to be quickly increased in each of the cases where following condition 1 is satisfied and following condition 2 is satisfied, and increases the output of heater wire H1 from the output in a normal time. Here, the normal time means a case where neither condition 1 nor condition 2 is satisfied. The output of heater wire H1 in the normal time includes 0 (zero). That is, an aspect of the present invention includes the case where heater wire H1 is energized only when following condition 1 is satisfied or when following condition 2 is satisfied, while otherwise is not energized in the normal time.

### (1) Condition 1

Based on the detection signal of door switch Sd, controller 100 has determined that outer door 3a is in the open state. Specifically, controller 100 has determined that culture space 20 is in a released state, because the detection signal of temperature sensor St indicated that the temperature in culture space 20 has decreased by a predetermined value or a value greater than the predetermined value while the controller has been determining, based on the detection signal of door switch Sd, that outer door 3a is in the open state. That is, it was presumed that air having a high humidity in culture space 20 has been discharged since culture space 20 has been in the released state and, instead, air around culture apparatus 1 having a relatively low humidity has flowed into culture space 20.

In the case where this condition 1 is satisfied and the output of heater wire H1 is controlled such that the output of heater wire H1 is higher than that in the normal time, controller 100 lowers the output of heater wire H1 back to the output in the normal time when a predetermined time elapses since it is determined, based on the detection signal of door switch Sd, that outer door 3a is switched to the closed state. Note that, controller 100 has a timer function, and counts the predetermined time using the timer function.

### (2) Condition 2

The predetermined time has not elapsed since controller 100 received the operation of turning on the power from operation device 50. That is, humidification of culture space 20 by the use of heater H1 has just been started and the humidity in culture space 20 is much lower than the appropriate range (range suitable for incubating a culture).

In the case where this condition 2 is satisfied and the output of heater wire H1 is controlled such that the output of heater wire H1 is higher than that in the normal time, controller 100 lowers the output of heater wire H1 back to the output in the normal time when a predetermined time elapses since controller 100 received the operation of turning on the power from operation device 50.

Note that, controller 100 may be configured to control the output of heater wire H1 such that the output of heater wire H1 is higher than that in the normal time when above-described condition 1 is satisfied, and set the output of heater wire H1 to the output in the normal time even when above-described condition 2 is satisfied. Conversely, controller 100 may be configured to set the output of heater wire H1 to the output in the normal time even when above-described condition 1 is satisfied, and control the output of heater wire H1 such that the output of heater wire H1 is higher than that in the normal time when above-described condition 2 is satisfied.

### [3. Effects]

According to an embodiment of the present invention, the following effects are obtained.
(1) Humidity adjustment heater wire H1 is provided as a part separate from temperature adjustment heater wire H2. It is thus possible to reduce the capacity of heater wire H1 as compared with the conventional technique in which a single heater wire serves as both a temperature adjustment heater wire and a humidity adjustment heater wire. Consequently, it is possible to control the temperature of humidity adjustment heater wire H1 more finely and with higher responsiveness than in the conventional technique. Thus, according to an embodiment of the present invention, the humidity in culture space 20 can be finely and quickly adjusted.
(2) Although dew condensation is likely to occur at a portion between bottom wall 2a1 of inner box 2a and humidification tray 6, humidity adjustment heater wire H1 is disposed below humidification tray 6, and it is thus possible to reduce the dew condensation at the portion effectively by heater wire H1.
(3) Humidification tray 6 is heated by a portion of temperature adjustment heater wire H2. Thus, the heating amount for adjusting the humidity in culture space 20 can be covered only by heater wire H2 in the normal time in which it is particularly unnecessary to increase the evaporation amount of humidification water W. Alternatively, it is possible to reduce the output of humidity adjustment heater wire H1 in the normal time.
(4) Since temperature adjustment heater wire H2 is also disposed below humidification tray 6, it is possible to effectively reduce the dew condensation at the portion between bottom wall 2a1 of inner box 2a and humidification tray 6 by heating the portion where the dew condensation is likely to occur.
(5) Temperature adjustment heater H2 to be steadily energized includes U-turn portion h20 disposed on center line CL of humidification tray 6 (or near center line CL) in plan view, U-turn portions h21 and h22 extending from the U-turn portion h20 to the left and right. Thus, the temperature adjustment heater is disposed with respect to humidification tray 6 without deviation, and it is possible to reduce the occurrence of a cold spot at which the temperature is locally low in humidification tray 6, so as to humidify culture space 20 stably and steadily.
   Further, U-turn portions h10, h11, h12, and h13 of humidity adjustment heater H1 are disposed to fill gaps between U-turn portions h20, h21, and h22 of temperature adjustment heater H2. It is thus possible to more effectively prevent the occurrence of the cold spot in humidification tray 6 so as to quickly increase the humidity in culture space 20 to the appropriate range.
(6) Controller 100 can control heater wires H1 and H2 individually. Thus, in order to increase the evaporation amount of humidification water W, only the output of heater wire H1 can be increased without changing the output of temperature adjustment heater wire H2. Accordingly, it is possible to adjust the humidity in culture space 20 while preventing disturbance to the temperature in culture space 20. Note that, when the output of heater wire H1 is increased, the output of heater wire H2 may be slightly decreased in order to further reduce the fluctuation (rise) in the temperature in culture space 20.
(7) On the condition that door switch Sd has detected the open state of outer door 3a, controller 100 increases the output of heater wire H1 to an output higher than that in the normal time (higher than an output during when door switch Sd detects the closed state of outer door 3a). Consequently, even when the humidity in culture space 20 rapidly decreases because outer door 3a and inner door 3b are opened, this decrease is cancelled out by an increase in the evaporation of humidification water W caused by an increase in the output of heater wire H1. It is thus possible to maintain the humidity in culture space 20 within the appropriate range.
(8) On the condition that a predetermined time has not elapsed since the operation of turning on the power was received from operation device 50, controller 100 increases the output of heater wire H1 to an output higher than the output in the normal time (the output after the predetermined time has elapsed since the operation of turning on the power was received). Consequently, when the humidity in culture space 20 has not yet been increased to the appropriate range because culture apparatus 1 has been just started, the humidity in culture space 20 can be increased to the appropriate range quickly.

### [4. Modifications]

(1) Temperature adjustment heater wire H2 may be removed from the lower surface of humidification tray 6, and humidification tray 6 may not be heated by heater wire H2. That is, humidification tray 6 may be heated only by humidity adjustment heater wire H1.
(2) The outputs of heaters H1 and H2 may be manually operated by an operator, either in conjunction with the control of controller 100 or in place of the control of controller 100.
(3) The pattern of heaters H1 and H2 is not limited to that illustrated in FIG. 2, and is appropriately changed depending on the size and shape of humidification tray 6.

The disclosure of Japanese Patent Application No. 2018-201702, filed on October 26, 2018, including the specification, claims, drawings and abstract is incorporated herein by reference in its entirety.

### Industrial Applicability

The present invention is suitably utilized as a culture apparatus.

### Reference Signs List

1 Culture apparatus
2 Heat insulation box
2a Inner box
2a1 Bottom wall
2b Outer box
2c Heat insulation material
2d Air layer
3 a Outer door
3b Inner door
4 Shelf
5 Duct
5a Suction port
5b Blow-out port
5c Circulation blower
6 Humidification tray
7 Ultraviolet lamp
10 Cover
11a Dew condensation member
12a, 12b Gas supply pipe
13 Electrical box
13a Inside of electrical box
20 Culture space
21 Opening
50 Operation device
100 Controller
c21, c10, c11, c20 Crimp terminal
CL Center line of humidification tray 6 in left-right direction
e10, e11, e20, e21 Power cable
H1 Humidity adjustment heater wire (first heater)
H2 Temperature adjustment heater wire (second heater)
h10, h11 U-turn portion (third portion)
h12, h13 U-turn portion (fourth portion)
h20 U-turn portion (first portion)
h21, h22 U-turn portion (second portion)
K Gas passage
P I, P2 Packing
S1 Space
S2 Mechanical room
St Temperature sensor
Sd Door switch
W Water

## Claims

1. A culture apparatus, comprising:
a humidification tray that is disposed in a culture space and stores a humidification liquid;
a first heater that heats the humidification tray; and
a second heater for controlling a temperature in the culture space, wherein
the first heater and the second heater are separately provided.

2. The culture apparatus according to claim 1, wherein
the first heater is disposed below the humidification tray.

3. The culture apparatus according to claims 1 or 2, wherein
a portion of the second heater heats the humidification tray.

4. The culture apparatus according to claim 3, wherein
the portion of the second heater is disposed below the humidification tray.

5. The culture apparatus according to claim 1, wherein
the first heater and a portion of the second heater are disposed below the humidification tray,
the portion of the second heater comprises:
a first portion disposed in a middle of the humidification tray in a first direction and extending in a second direction crossing the first direction, and
a plurality of second portions extending from the first portion in the first direction and disposed to be spaced apart from one another in the second direction,
and
the first heater comprises:
third portions being two in number, the third portions being disposed on opposite outer sides of the portion of the second heater in the first direction and extending in the second direction, and
a fourth portions extending from the third portions in the first direction and disposed between the plurality of second portions.

6. The culture apparatus according to any one according to claims 1 to 5, further comprising:
a controller that controls an output of the first heater and an output of the second heater individually.

7. The culture apparatus according to claim 6, further comprising:
a door that opens and closes the culture space; and
a detection device that detects opening and closing of the door, wherein
the controller controls the output of the first heater based on a detection result of the detection device.

8. The culture apparatus according to claim 7, wherein,
on condition that the controller determines that the door is in an open state based on the detection result of the detection device, the controller controls the output of the first heater such that the output of the first heater is higher than that in a case where the door is determined to be in a closed state based on the detection result.

9. The culture apparatus according to any one according to claims 6 to 8, wherein
the controller controls the output of the first heater, until a predetermined time elapses after power of the culture apparatus is turned on, such that the output of the first heater is higher than that after the predetermined time elapses.
